(19) Europäisches Patentamt
European Patent Office
Office européen des brevets

(11) EP 4 674 401 A2

(12) **EUROPEAN PATENT APPLICATION**

(43) Date of publication:
**07.01.2026 Bulletin 2026/02**

(21) Application number: **25217166.5**

(22) Date of filing: **25.12.2020**

(51) International Patent Classification (IPC):
***A61J 1/14*** *(2023.01)*

(52) Cooperative Patent Classification (CPC):
**A61P 27/02; A61K 9/0048; A61K 9/08; A61K 31/519; A61K 47/02**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.12.2019 JP 2019239599**

(62) Document number(s) of the earlier application(s) in accordance with Art. 76 EPC:
**20907783.3 / 4 070 796**

(71) Applicant: **Rohto Pharmaceutical Co., Ltd**
**Osaka-shi**
**Osaka 544-8666 (JP)**

(72) Inventors:
- **NISHIMOTO, Akinori**
  **Osaka-shi, 544-8666 (JP)**
- **KITA, Akiko**
  **Osaka-shi, 544-8666 (JP)**
- **HAYASHI, Saeko**
  **Osaka-shi, 544-8666 (JP)**

(74) Representative: **Mewburn Ellis LLP**
**Aurora Building**
**Counterslip**
**Bristol BS1 6BX (GB)**

Remarks:
This application was filed on 20-11-2025 as a divisional application to the application mentioned under INID code 62.

(54) **CONTAINER ACCOMMODATING AN AQUEOUS OPHTHALMIC COMPOSITION COMPRISING DELGOCITINIB**

(57) The present invention relates to an aqueous composition comprising delgocitinib or a salt thereof and having a pH of 4.0 to 6.5.

EP 4 674 401 A2

## Description

### Technical Field

The present invention relates to an aqueous composition.

### Background Art

Janus kinase (JAK) is a non-receptor tyrosine kinase that plays an important role in intracellular immunologically activated signal transduction, and drugs having Janus kinase inhibitory activity are expected to improve autoimmune diseases and allergic diseases through suppression of excessive activation of the immune response. As one of the compounds having an inhibitory effect of Janus kinase, 3-[(3S,4R)-3-methyl-6-(7H-pyrrolo[2,3-d]pyrimidin-4-yl)-1,6-diazaspir o[3,4]octan-1-yl]-3-oxopropanenitrile (generic name: delgocitinib) is known (for example, Patent Literature 1).

### Citation List

### Patent Literature

[Patent Literature 1]
International Publication No. WO 2017/006968

### Summary of Invention

### Technical Problem

In the case of using delgocitinib as a therapeutic agent for diseases in the ophthalmic field, the ophthalmic preparation containing delgocitinib is required to have a certain degree of stability.
An object of the present invention is to provide an aqueous composition with excellent stability, containing delgocitinib or a salt thereof as an active ingredient.

### Solution to Problem

As a result of diligent studies to solve the problem, the present inventor has found that the stability of the aqueous composition is remarkably improved by controlling the pH of the aqueous composition containing delgocitinib to a specific range. The present invention is based on the finding and each of the following inventions is provided.

[1] An aqueous composition comprising delgocitinib or a salt thereof and having a pH of 4.0 to 6.5.
[2] The aqueous composition according to item [1], wherein the aqueous composition is accommodated in a container having a portion in contact with the aqueous composition partly or entirely formed of a polyolefin-based resin.
[3] The aqueous composition according to item [1] or [2], wherein the content of delgocitinib or a salt thereof is 0.001 mass% to 5 mass% based on the total amount of the aqueous composition.
[4] The aqueous composition according to item [2] or [3], wherein the polyolefin-based resin is polyethylene.
[5] The aqueous composition according to any one of items [1] to [4], further comprising a buffer.
[6] The aqueous composition according to any one of items [1] to [5], wherein the aqueous composition is for ophthalmology.

### Advantageous Effect of Invention

According to the present invention, an aqueous composition with excellent stability, containing delgocitinib or a salt thereof as an active ingredient, can be provided.

### Description of Embodiments

Hereinafter, embodiments of the present invention will be described in detail. The present invention, however, is not limited to the following embodiments.
The aqueous composition according to the present embodiment contains delgocitinib or a salt thereof.
Delgocitinib is also referred to as 3-[(3S,4R)-3-methyl-6-(7H-pyrrolo[2,3-d]pyrimidin-4-yl)-1,6-diazaspir o[3,4] octan-1-yl]-3-oxopropanenitrile, which is a known compound represented by the following formula:

[Chemical Formula 1]

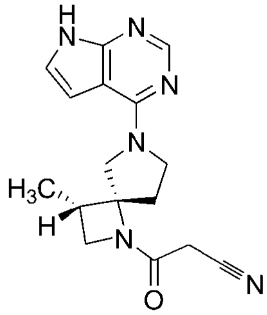

Delgocitinib or a salt thereof may be produced, for example, by the method described in International Publication No. WO 2017/006968 or International Publication No. WO 2018/117151.

The salt of delgocitinib is not particularly limited as long as it is pharmaceutically, pharmacologically (in drug manufacturing) or physiologically acceptable. Specific examples of the salts include salts with an inorganic acid, salts with an organic acid, salts with an inorganic base, salts with an organic base, salts with an acidic amino acid, and salts with a basic amino acid.

Examples of the salts with an inorganic acid include salts with hydrochloric acid, hydrobromic acid, sulfuric acid, nitric acid, or phosphoric acid. Examples of the salts with an organic acid include salts with acetic acid, succinic acid, fumaric acid, maleic acid, tartaric acid, citric acid, lactic acid, stearic acid, benzoic acid, methanesulfonic acid (mesylic acid), ethanesulfonic acid, or p-toluenesulfonic acid. Examples of the salts with an inorganic base include alkali metal salts such as a sodium salt and a potassium salt, alkaline earth metal salts such as a calcium salt and a magnesium salt, and an aluminum salt and an ammonium salt. Examples of the salts with an organic base include salts with diethylamine, diethanolamine, meglumine, or N,N-dibenzylethylenediamine. Examples of the salts with an acidic amino acid include salts with aspartic acid, or glutamic acid. Examples of the salts with a basic amino acid include salts with arginine, lysine, or ornithine.

The aqueous composition according to the present embodiment contains delgocitinib or a salt thereof as an active ingredient, and may be used for the treatment of corneal epithelium disorder caused by an intrinsic disease such as dry eye (xerophthalmia syndrome), Sjogren's syndrome, and Stevens-Johnson syndrome, or corneal epithelium disorder caused by exogenous diseases resulting from post-operation, drug-induction, external injury, or contact lens wearing.

The aqueous composition according to the present embodiment promotes tear secretion due to containing delgocitinib or a salt thereof, and therefore may be used for improving dry eye symptoms. The dry eye may be a dry eye caused by autoimmune diseases such as Sjogren's syndrome, or may be a dry eye caused by a factor other than autoimmune diseases.

The content of delgocitinib or a salt thereof in the aqueous composition according to the present embodiment is not particularly limited, and is appropriately set according to the type and content of other compounding components, formulation form, and the like. From the viewpoint of exerting the effect of the present invention more remarkably, the content of delgocitinib or a salt thereof is as follows. For example, based on the total amount of the aqueous composition according to the present embodiment, the total content of delgocitinib or a salt thereof is preferably 0.001 mass% to 5 mass%, more preferably 0.003 mass% to 3 mass%, still more preferably 0.005 mass% to 1 mass%, furthermore preferably 0.01 mass% to 0.5 mass%, particularly preferably 0.015 mass% to 0.4 mass%, and particularly more preferably 0.02 mass% to 0.3 mass%.

[Buffer]

The aqueous composition according to the present embodiment may further contain a buffer. The aqueous composition further containing a buffer allows the effect of the present invention to be more remarkably exhibited. The buffer is not particularly limited as long as it is pharmaceutically, pharmacologically (in drug manufacturing) or physiologically acceptable.

Examples of the buffer include a boric acid buffer (for example, boric acid and a combination of boric acid and borax). As the buffer, a commercially available one may be used. The buffer may be used alone or in combination of two or more. Boric acid is preferred as the buffer.

The content of the buffer in the aqueous composition according to the present embodiment is not particularly limited, and is appropriately set according to the type of the buffer, the type and content of other compounding components, the use and formulation form of the aqueous composition, etc. From the viewpoint of exerting the effect of the present

invention more remarkably, the content of the buffer is as follows. For example, the total content of the buffer based on the total amount of the aqueous composition is preferably 0.01 mass% to 10 mass%, more preferably 0.05 mass% to 5 mass%, and still more preferably 0.1 mass% to 3 mass%.

The content ratio of the buffer to delgocitinib or a salt thereof in the aqueous composition according to the present embodiment is not particularly limited, and set appropriately according to the type of buffer, the type and content of other compounding components, the use and formulation form of the aqueous composition, etc. From the viewpoint of further enhancing the effect of the present invention, the content ratio of the buffer relative to delgocitinib or a salt thereof is as follows. For example, the total content of the buffer relative to 1 part by mass of the total content of delgocitinib or a salt thereof in the aqueous composition according to the present embodiment is preferably 0.03 parts by mass to 500 parts by mass, more preferably 0.1 parts by mass to 250 parts by mass, and still more preferably 0.3 parts by mass to 150 parts by mass.

[Inorganic salt]

The aqueous composition according to the present embodiment may further contain an inorganic salt. The aqueous composition further containing an inorganic salt allows the effect of the present invention to be more remarkably exhibited. The inorganic salt is not particularly limited as long as it is pharmaceutically, pharmacologically (in drug manufacturing) or physiologically acceptable.

Examples of the inorganic salts include chloride salts such as sodium chloride, potassium chloride, calcium chloride, and magnesium chloride. Commercially available inorganic salts may be used. As the inorganic salts, one type may be used alone, or two or more types may be used in combination. As the inorganic salts, sodium chloride and potassium chloride are preferred.

The content of the inorganic salts in the aqueous composition according to the present embodiment is not particularly limited, and set appropriately according to the type of inorganic salt, the type and content of other compounding components, the use and formulation form of the aqueous composition, etc. From the viewpoint of exerting the effect of the present invention more remarkably, the content of the inorganic salts is as follows. For example, the total content of the inorganic salts based on the total amount of the aqueous composition is preferably 0.00001 mass% to 3 mass%, more preferably 0.0001 mass% to 2 mass%, and still more preferably 0.001 mass% to 1.5 mass%.

The pH of the aqueous composition according to the present embodiment is 4.0 to 6.5. With a pH of the aqueous composition controlled to the range, the stability of the aqueous composition containing delgocitinib or a salt thereof as an active ingredient is remarkably improved. From the viewpoint of further remarkably improving the stability of the aqueous composition, the pH of the aqueous composition is preferably 4.5 to 6.5, 4.5 to 6.0 or 5.0 to 6.5, and more preferably 5.0 to 6.0.

The aqueous composition according to the present embodiment may be adjusted to an osmotic pressure ratio within a range acceptable to a living body on an as needed basis. The suitable osmotic pressure ratio may be appropriately set depending on the use, the formulation form, the usage method, etc. of the aqueous composition, and for example, may be set to 0.4 to 5.0, preferably 0.6 to 3.0, more preferably 0.8 to 2.2, and still more preferably 0.8 to 2.0. The osmotic pressure ratio is the ratio of osmotic pressure of a sample to 286 mOsm (osmotic pressure of 0.9 w/v% sodium chloride aqueous solution) based on Japanese Pharmacopoeia 17th edition, and the osmotic pressure is measured with reference to the osmotic pressure measurement method described in Japanese Pharmacopoeia (cryoscopic method). The standard solution for measuring the osmotic pressure ratio (0.9 w/v% sodium chloride aqueous solution) may be prepared by drying sodium chloride (Japanese Pharmacopoeia standard reagent) at 500 to 650°C for 40 to 50 minutes, then cooling it in a desiccator (silica gel), precisely weighing 0.900 g thereof, and dissolving it in purified water to prepare exactly 100 mL, or by using a commercially available standard solution for measuring osmotic pressure ratio (0.9 w/v% sodium chloride aqueous solution).

The viscosity of the aqueous composition according to the present embodiment is not particularly limited as long as it is pharmaceutically, pharmacologically (in drug manufacturing) or physiologically acceptable. As the viscosity of the aqueous composition according to the present embodiment, for example, the viscosity at 20°C measured with a rotational viscometer (RE550 type viscometer, manufactured by Toki Sangyo Co., Ltd., rotor; 1°34'×R24) is preferably 0.5 to 10 mPa·s, more preferably 1 to 5 mPa·s, and still more preferably 1 to 3 mPa·s.

The aqueous composition according to the present embodiment may be prepared, for example, by adding and mixing a desired content of delgocitinib or a salt thereof and, on an as needed basis, other components. Specifically, for example, the preparation is performed by dissolving or suspending the components in purified water and sterilizing the liquid by filtration sterilization or the like.

The aqueous composition according to the present embodiment may be in various dosage forms depending on the purpose, and examples thereof include liquid preparations, gel preparations, and semi-solid preparations (ointments, etc.).

The aqueous composition according to the present embodiment may be used for ophthalmology. Further, the

aqueous composition according to the present embodiment may be used, for example, as eye drops (also referred to as an eye wash or an ophthalmic solution; eye drops include an artificial tear solution and eye drops that can be instilled while wearing contact lenses).

In the case where the aqueous composition according to the present embodiment is an eye drop, the dosage and administration thereof is not particularly limited as long as it is effective and has few side effects, and examples thereof include, for adults (15 years old or older) and children 7 years old or older, 1 drop or 1 to 2 drops in an eye at a time, 4 times a day, and 1 drop or 1 to 2 drops in an eye at a time, 5 to 6 times a day.

(Polyolefin-based resin container)

It is preferable that the aqueous composition according to the present embodiment be provided in a container having a portion in contact with the aqueous composition partly or entirely formed of a polyolefin-based resin (also simply referred to as "polyolefin-based resin container"). The polyolefin-based resin container may be a package having a portion in contact with the aqueous composition, and may include, for example, a container main body portion for accommodating the aqueous composition, a portion including a discharge portion of the container (for example, a nozzle, an inner plug), a suction tube, a cap, etc.

The polyolefin-based resin may be any of a polymer obtained by polymerizing one type of olefin alone and a polymer obtained by copolymerizing two or more types of olefins. These polymers may contain other polymerizable monomers as constituent components. Specific examples of the polyolefin-based resin include polyethylene (including low-density polyethylene, medium-density polyethylene, high-density polyethylene, etc.), polypropylene (including isotactic polypropylene, syndiotactic polypropylene, atactic polypropylene, etc.), and an ethylene-propylene copolymer and polymethylpentene. Among these, polyethylene and polypropylene are preferred, and polyethylene is more preferred.

The polyolefin-based resin container that accommodates the aqueous composition may be a container generally used in the field of ophthalmology, and specifically, for example, an eye drop container or an eyewash container. It is preferable that the type of container be an eye drop container

The polyolefin-based resin container according to the present embodiment has a portion in contact with the aqueous composition partly or entirely formed of a polyolefin-based resin. Examples of the portion in contact with the aqueous composition in the polyolefin-based resin container for accommodating the aqueous composition include an accommodating portion (in the case where the container has a structure including a plurality of layers, the innermost layer), an inner plug, and a perforated inner plug. For example, in the case where the polyolefin-based resin container has a perforated inner plug (nozzle), the accommodating portion of the aqueous composition other than the perforated inner plug may be formed of a polyolefin-based resin, or the entire container may be made of a polyolefin-based resin. The polyolefin-based resin container according to the present embodiment may have a portion in contact with the aqueous composition partly formed of polyolefin-based resin, and it is preferable that the accommodating portion be formed of polyolefin-based resin from the viewpoint of exerting the effect of the present invention more remarkably.

The shape and capacity of the polyolefin-based resin container according to the present embodiment are not particularly limited, and may be appropriately set according to the intended use. In the case where the polyolefin-based resin container is a container for accommodating eye drops, the volume may be, for example, 0.3 mL or more and 25 mL or less, preferably 1 mL or more and 10 mL or less, and more preferably 2 mL or more and 7 mL or less.

The polyolefin-based resin container according to the present embodiment may be a multi-dose type that accommodates an amount used multiple times, or may be a unit-dose type that accommodates an amount used single time. In the case where the polyethylene-based resin container is a unit dose type, the volume may be, for example, 0.1 mL or more and 1 mL or less, and preferably 0.2 mL or more and 0.5 mL or less.

## Examples

Hereinafter, the present invention will be specifically described based on test examples, though the present invention is not limited thereto. Unless otherwise specified, the unit of each component in the tables is w/v%.

[Test Example 1: Stability test (1)]

Aqueous compositions were prepared according to a conventional method with the compositions shown in Table 1. Each of the prepared aqueous compositions was filtered through a 0.2-μm membrane filter and sterilized. Then, the aqueous composition was filled in an eye drop bottle (material: polyethylene, volume: 5 mL) and stored at 50°C for 2 months under light-shielding conditions. The content of delgocitinib in each of the preparation immediately after preparation and the preparation after storage was determined by the HPLC method (under measurement conditions described below, using measurement conditions 1 or 2), and according to the following formula 1, the delgocitinib residual ratio was calculated. The results are shown in Table 1.

Delgocitinib residual ratio (%) = (Content of delgocitinib after storage at 50°C for 2 months/Content of delgocitinib immediately after preparation × 100) - Moisture permeability [Formula 1]

In the formula 1, "moisture permeability" was calculated according to the following formula 2.

Moisture permeability (%) = (Weight of product filled in immediately after preparation - Weight of product filled in after storage at 50°C for 2 months)/(Weight of product filled in immediately after preparation - Empty weight of eye drop bottle) × 100 [Formula 2]

(HPLC measurement conditions 1)

Detector: ultraviolet absorptiometer (measurement wavelength: 254 nm)
Column: Inertsil ODS-2 (inner diameter: 4.6 mm, length: 150 mm, particle size: 5 μm)
Column temperature: constant temperature around 40°C
Mobile phase: solution of 1.50 g of sodium lauryl sulfate dissolved in 1000 mL of acetonitrile/diluted acetic acid (100) (1→60) mixture (7:3)
Flow rate: 0.9 mL/min

(HPLC measurement conditions 2)

Detector: Ultraviolet absorptiometer (measurement wavelength: 283 nm)
Column: Inertsil Ph-3 (inner diameter: 4.6 mm, length: 150 mm, particle size: 5 μm)
Column temperature: Constant temperature around 40°C
Mobile phase: 1000 mL of liquid prepared by adding 3.9 g of ammonium acetate and 12.5 mL of acetic acid (100) to water /acetonitrile mixture (4:1)
Flow rate: Approximately 0.8 mL/min

[Table 1]

| | Example 1 | Example 2 | Example 3 | Comparative Example 1 | Example 4 | Example 5 | Example 6 | Comparative Example 2 |
|---|---|---|---|---|---|---|---|---|
| Delgocitinib | 0.02 | 0.02 | 0.02 | 0.02 | 0.3 | 0.3 | 0.3 | 0.3 |
| Boric acid | 1.5 | 1.5 | 1.5 | 1.5 | 1.5 | 1.5 | 1.5 | 1.5 |
| Sodium chloride | 0.4 | 0.4 | 0.4 | 0.4 | 0.2 | 0.2 | 0.2 | 0.2 |
| Potassium chloride | 0.1 | 0.1 | 0.1 | 0.1 | 0.03 | 0.03 | 0.03 | 0.03 |
| Hydrochloric acid/sodium hydroxide | Appropriate amount | Appropriate amount | Appropriate amount | Appropriate amount | Appropriate amount | Appropriate amount | Appropriate amount | Appropriate amount |
| Purified water | Residual amount | Residual amount | Residual amount | Residual amount | Residual amount | Residual amount | Residual amount | Residual amount |
| pH | 5.0 | 5.5 | 6.0 | 7.0 | 5.0 | 5.5 | 6.0 | 7.0 |
| Residual ratio of delgocitinib (%) | 96.0 | 96.5 | 96.0 | 89.9 | 98.3 | 97.7 | 97.8 | - |

In Comparative Example 2, delgocitinib was not dissolved.

[Test Example 2: Stability test (2)]

Aqueous compositions were prepared according to a conventional method with the compositions shown in Table 2. Each of the prepared aqueous compositions was filtered through a 0.2-μm membrane filter and sterilized. Then, the aqueous composition was filled in an eye drop bottle (material: polyethylene, volume: 5 mL) and stored at 40°C for 5 months under light-shielding conditions. The content of delgocitinib contained in each of the preparation immediately after preparation and the preparation after storage was determined by the HPLC method (under the same measurement conditions in Test Example 1), and according to the following formula 3, the delgocitinib residual ratio was calculated. The results are shown in Table 2.

Delgocitinib residual ratio (%) = (Content of delgocitinib after storage at 40°C for 5 months/Content of delgocitinib immediately after preparation × 100) - Moisture permeability [Formula 3]

In the formula 3, "moisture permeability" was calculated according to the following formula 4.

Moisture permeability (%) = (Weight of product filled in immediately after preparation - Weight of product filled in after storage at 40°C for 5 months)/(Weight of product filled in immediately after preparation - Empty weight of eye drop bottle) × 100 [Formula 4]

[Table 2]

| | Example 7 | Example 8 | Comparative Example 3 | Example 9 | Example 10 | Example 11 |
|---|---|---|---|---|---|---|
| Delgocitinib | 0.02 | 0.02 | 0.02 | 0.3 | 0.3 | 0.3 |
| Boric acid | 1.5 | 1.5 | 1.5 | 1.5 | 1.5 | 1.5 |
| Sodium chloride | 0.4 | 0.4 | 0.4 | 0.2 | 0.2 | 0.2 |
| Potassium chloride | 0.1 | 0.1 | 0.1 | 0.03 | 0.03 | 0.03 |
| Hydrochloric acid/sodium hydroxide | Appropriate amount | Appropriate amount | Appropriate amount | Appropriate amount | Appropriate amount | Appropriate amount |
| Purified water | Residual amount | Residual amount | Residual amount | Residual amount | Residual amount | Residual amount |
| pH | 4.0 | 6.0 | 7.0 | 4.0 | 5.0 | 6.0 |
| Residual ratio of delgocitinib (%) | 98.1 | 96.6 | 93.9 | 99.8 | 99.1 | 97.7 |

[Test Example 3: Stability test (3)]

Aqueous compositions were prepared according to a conventional method with the compositions shown in Table 3. Each of the prepared aqueous compositions was filtered through a 0.2-μm membrane filter and sterilized. Then, 5 mL of the aqueous composition was filled in a centrifuge tube (material: polypropylene, volume: 15 mL) and stored at 50°C for 1 month under light-shielding conditions. The content of delgocitinib contained in each of the preparation immediately after preparation and the preparation after storage was determined by the HPLC method (under the same measurement conditions as in Test Example 1), and according to the following formula 5, the delgocitinib residual ratio was calculated. The results are shown in Table 3.

Delgocitinib residual ratio (%) = (Content of delgocitinib after storage at 50°C for 1 month/Content of delgocitinib immediately after preparation × 100) - Moisture permeability [Formula 5]

In the formula 5, "moisture permeability" was calculated according to the following formula 6.

Moisture permeability (%) = (Weight of product filled in immediately after preparation - Weight of product filled in after storage at 50°C for 1 month)/(Weight of product filled in immediately after preparation - Empty weight of eye drop bottle) $\times$ 100 [Formula 6]

[Table 3]

| | Example 12 | Example 13 | Example 14 |
|---|---|---|---|
| Delgocitinib | 0.3 | 0.3 | 0.3 |
| Boric acid | 1.5 | 1.5 | 1.5 |
| Sodium chloride | 0.2 | 0.2 | 0.2 |
| Potassium chloride | 0.03 | 0.03 | 0.03 |
| Hydrochloric acid/sodium hydroxide | Appropriate amount | Appropriate amount | Appropriate amount |
| Purified water | Residual amount | Residual amount | Residual amount |
| pH | 4.0 | 5.0 | 6.0 |
| Residual ratio of delgocitinib (%) | 98.4 | 98.4 | 97.5 |

## Claims

1. An aqueous ophthalmic composition comprising delgocitinib or a salt thereof and having a pH of 4.0 to 6.5.

2. The aqueous ophthalmic composition according to claim 1, wherein the aqueous ophthalmic composition is accommodated in a container having a portion in contact with the aqueous ophthalmic composition partly or entirely formed of a polyolefin-based resin.

3. The aqueous ophthalmic composition according to claim 1 or 2, wherein a content of delgocitinib or a salt thereof is 0.001 mass% to 5 mass% based on a total amount of the aqueous ophthalmic composition.

4. The aqueous ophthalmic composition according to claim 2 or 3, wherein the polyolefin-based resin is polyethylene.

5. The aqueous ophthalmic composition according to any one of claims 1 to 4, further comprising a buffer.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017006968 A
- WO 2018117151 A


### Non-patent literature cited in the description


- Japanese Pharmacopoeia